# EUROPEAN PATENT SPECIFICATION

(11) **EP 1 646 361 B1**
(45) Date of publication and mention of the grant of the patent: **22.12.2010**
(21) Application number: 04730363.1
(22) Date of filing: 29.04.2004
(51) Int. Cl.: A61K 8/55, A61K 8/63, A61Q 19/08

(54) **EMULSIFIER SYSTEM BASED ON VEGETABLE LIPOPROTEINS IN COMBINATION WITH PHYTOSTEROLS AND/OR VEGETABLE LECITHINS AND THEIR USE IN COSMETIC AND PHARMACEUTICAL EMULSIONS**
EMULGIERMITTELSYSTEM AUF DER BASIS VON PFLANZLICHEN LIPOPROTEINEN IN KOMBINATION MIT PHYTOSTEROLEN UND/ODER PFLANZLICHEN LECITHINEN UND IHRE VERWENDUNG IN KOSMETISCHEN UND PHARMAZEUTISCHEN EMULSIONEN
SYSTEME D'EMULSIFIANTS A BASE DE LIPOPROTEINES VEGETALES COMBINEES A DES PHYTOSTEROLS ET/OU A DES LECITHINES VEGETALES ET SON UTILISATION DANS DES EMULSIONS COSMETIQUES ET PHARMACEUTIQUES

(30) Priority: 30.04.2003 IT MI20030884
(43) Date of publication of application: 19.04.2006
(73) Proprietor: Bottega Verde S.r.l., 53026 Pienza (SI) (IT); Maycos Italiana S.A.S., 46043 Castiglione delle Stiviere (MN) (IT)
(72) Inventor: GHIRARDINI, Adriano, I-48100 Ravenna (IT); COMINI, Miro, I-46043 Castiglione delle Stiviere (MN) (IT)
(74) Representative: Pipparelli, Claudio
(86) International application number: PCT/IT2004/000236
(87) International publication number: WO 2004/096170

(56) References cited:
- WO-A-97/14713
- WO-A-02/062304
- FR-A- 2 520 253
- GB-A- 2 047 563
- US-A- 5 458 881
- US-A- 5 762 916

## Description

The present invention relates to compositions of emulsifiers containing lipoproteins to be used in the pharmaceutical and cosmetic fields: more specifically, the present invention relates to compositions of emulsifiers comprising at least one lipoprotein and at least a second component selected from phytosterols and phospholipids.

It is well known that an emulsion is a more or less stable dispersion of a liquid, in the form of very fine drops (dispersed phase) in another non-miscible liquid (dispersing phase). Its stability depends on several factors, such as, for example, the density of the two phases, the temperature and the presence of stabilizing substances, called emulsifiers, which, according to the physico-chemical principles that regulate the behaviour of surfactants, are oriented towards the interface phase, thus reducing its free energy and allowing, in such a way, a more or less high dispersion degree.

With particular reference to the cosmetic field, the increasing importance of the appearance, structure and functionality of an emulsion, is known.

However, the more and more frequent use of synthesis emulsifiers often leads to skin damage with the result that, while, on the one hand, more and more active ingredients are added, on the other hand, the frequent use of base synthesis emulsifiers reduces their efficacy, with a negative influence on the skin tolerance of the cosmetic product.

It is known that in the human hydro-lipid mantle for skin defense, systems are included which allow aggregation states of lipids with water, proteins, lipoproteins, amino acids, mono-, di-, tri-glycerides, cholesterol. These substances are part of the cellular membrane, in which their aggregation allows the structure.

The cell membrane and the skin hydro-lipid mantle are aggregation states defined by lipids with hydrophilic substances.

This natural, spontaneous aggregation system is known as the liposome system.

Liposomes are micro or nano emulsions of hydro-lipid bodies having a very fine structure, obtained from natural substances, phospholipids, lipoproteins, sterols, etc, with an affinity for the skin, completely tolerated and also used in pharmacology as carriers of active substances. Liposomes have been used in the cosmetic field as such or added to pre-prepared emulsions. However, the liposomes of emulsions obtained by means of synthesis emulsifiers are deactivated sine their structure is disintegrated by the main structure, having high chemical energy, of the synthesis emulsion. Liposomes were manufactured by using a low lipid content and have never been intended with a high content of the fatty phase, as in the case of cosmetic emulsions.

Emulsifier composition of natural origin are known from the following documents :

US-A-5 458 881 discloses an emulsifier system based on :
A) at least one vegetable lipoprotein in combination with
B) a phytosterol and/or
C) a phospholipid of vegetable origin.

This document provides also a cosmetic emulsion comprising:
D) a vegetable lipoprotein (capryloyl derivate of wheat protein amino acids) and
E) Cholecithin.

FR-A-2 520 253 discloses an emulsifying system based on:
1) an animal lipoprotein,
2) a phytosterol;
3) a vegetable phosphatide.

GB-A-2 047 563 describes an emulsifying system comprising:
A) the condensation product of a fatty acid and an amino acid;
B) a phytosterol.

Additionally a phospholipids component can also be present in the emulsifying system described in this document.

US-A-5762916 discloses a cosmetic or pharmaceutical composition containing an emulsifier comprising a highly acylated protein fatty acid condensate having a total nitrogen content based on the acylation product of 1.8 to 4.1% by weight. WO-A-9714713 describes amphiphilic complexes produced by reacting one or more proteins having an average molecular weight of at least 5000 Daltons with one or more C₄-C₃₀ fatty chains selected from fatty acids, fatty alcohols, fatty amines and derivatives thereof, at a temperature between room temperature and 80°C, the weight ratio of the reagents [protein(s):fatty acid(s)] being from 1/1 to 1/10.

The present patent application defines a composition of emulsifiers which can be used in both the cosmetic and pharmaceutical field, comprising constituents of a natural origin which overcome all the drawbacks of the known products, thus offering a considerable contribution from a dermatological point of view, and consequently representing significant progress in that, so far, particularly in the cosmetic field, the used active emulsifier has always been a synthesis product and, together with perfumes and preservatives, potentially represents the sensitivity and allergenic element for skin incompatibility.

An object of the present invention therefore relates to a composition of emulsifiers, which can be used as an emulsifying base for cosmetic and pharmaceutical products, comprising at least one vegetable lipoprotein and at least a second component selected from phytosterols and phospholipids, these substances also being of a strictly vegetable origin.

The present application defines a composition of emulsifier for cosmetic and pharmaceutical products comprising:
(a) a first component selected from the group consisting of lipoproteins of almond, rice, wheat, said lipoproteins being the reaction product of fatty acids and hydrolysed proteins, and
(b) a second component selected from the group consisting of phytosterols and vegetable phospholipids.

The cosmetic/farmaceutical products prepared through the compositions of the present invention have the following characteristics:
- the pH value is similar to that of the hydrolipid skin mantle;
- the primary emulsifiers have an exceptional skin compatibility;
- the emulsions are rapidly absorbed by the skin, leaving it smooth, soft, elastic and hydrated;
- the emulsions are very brilliant and stable to thermal shock;
- the emulsions are an ideal carrier for further skin active ingredients;
- they are compatible with all the lipids commonly used in cosmetics.

In addition to the primary constituents, lipoproteins and phytosterols, and/or phospholipids, the compositions of emulsifiers according to the present invention might comprise one or more of the following substances: vegetable monoglycerides and fatty alcohols.

Among lipoproteins, the use of those of rice, almonds and wheat is of great interest. The phytosterols can be, in turn, preferably selected from those of soybean, rape, and rice (gamma oryzanol) while the phosphatides (phospholipids) can be preferably of soybean and rice. Through the above compositions of emulsifiers, stable emulsions are easily obtained for the production of cosmetics for body and skin care, having an absolute skin compatibility and cosmetic activity in the skin thickening and consequently in its defense against outside exogenous aggression and solar UVB ray irradiations. Cleansing milks, softening hydrating and anti-age creams and sun protection emulsions can therefore be easily manufactured. These compositions of emulsifiers can be used with absolute safety, from toxicological and allergological point of view, as their components are already present in nature.

In the case of the use of substances belonging to the group of lipoproteins and phytosterols, the use of a lipoprotein of almonds or of rice, is particularly significant. Almond lipoproteins are obtained by reacting fatty acids of vegetable oils, wherein palmitic acid, stearic acid, palm coconut and almond oleic acid, laurylic, miristic and palmitoleic acid of vegetable oils are more important than others.

These fatty acids are reacted with an almond and/or rice hydrolyzed protein.

Phytosterols of soybean and rape and rice (gamma oryzanol), for example, which are also particularly effective, can be found on the market.

An almond lipoprotein can be obtained through the reaction of palmitic acid by the acylation of its chloride with a hydrolyzed protein product of the almond, its potassium salt, in aqueous solution at 70-80°C for at least two hours. The thus obtained lipoprotein is subsequently acidified in order to separate it from the reaction salts and from the water mass. The lipoprotein obtained is of acid nature and has an active content of between 60-80%. The protein of the hydrolyzed almond has an active content of 10-30%, is amber-coloured and a limpid liquid whose amino acid composition is as follows:

| CySO₃H | 0.75 mol% | (CYS)₂ | 0.62 mol% |
|---|---|---|---|
| ASP | 9.45 | MET | 0.33 |
| THR | 3.64 | ILE | 0.99 |
| SER | 4.24 | LEU | 2.01 |
| GLU | 27.94 | TYR | 0.89 |
| PRO | 4.70 | PHE | 1.39 |
| GLY | 11.29 | ORN | 0.90 |
| ALA | 6.40 | LYS' | 2.89 |
| VAL | 3.09 | HIS | 1.75 |
| ARG | 7.52 | | |

A rice lipoprotein is obtained by reacting palmitic acid and/or coconut fatty acids by the acylation of their chloride with a rice hydrolyzed protein product, its potassium and/or sodium salt, in aqueous solution at 70-80°C for at least two hours.

The obtained lipoprotein is then acidified in order to separate it from the reaction salts and water. The obtained lipoprotein is of acidic nature and has an active titer of between 60-80%. The rice hydrolyzed protein product has an active titer of 10-30%, is amber-coloured and is a limpid liquid whose amino acid composition is as follows:

| AMINO ACID PROFILE: | | | |
|---|---|---|---|
| ALA | 8 mol % | MET | 1.8 mol % |
| ARG | 9.7 | PHE ALAPRO | 5.7 |
| ASP | 8.6 | SER | 4.2 |
| CYS | 1,1 | THR | 5.2 |
| GLU | 17.6 | TRY | 4.4 |
| CLY | 4.1 | TYR | 1.0 |
| HIS | 2.9 | VAL | 5.2 |
| ILE | 4.9 | | 6.6 |
| LEU | 7.7 | | |
| LIS | 4.2 | | |

In the case of a composition comprising a phoshatide, the use of phosphatyl choline (lecithin) in combination with the hydrolyzed palmitic potassium of rice or almond protein, is quite interesting.

Both substances have poor free chemical energy (they do not significantly reduce the water surface tension and water/oil interface tension). They are the main component of the hydrolipid skin defense barrier.

### EXAMPLE 1

| (preferred) | | |
|---|---|---|
| 1. Palmitoyl Hydrolyzed Sweet Almond Protein 75% S.A. | 10-50% | 25% |
| 2. Rape Vegetable Phytosterols | 10-50% | 15% |
| 3. Glyceryl Stearate | 20-40% | 26.25% |
| 4. Cetearyl Alcohol | 20-40% | 26.25% |
| 5. Potassium Hydroxide 50% | 5-15% | 7.5% |

262.5 g of glyceryl stearate and 262.5 g of cetearyl alcohol are melted at 80°C in a Pyrex glass until the product is completely liquid. 150 g of phytosterols are added at the same temperature, and the temperature is then increased to 105°C, the whole mixture is stirred until a complete solution is obtained, it is then cooled, still under stirring, to 80°C. At this point, 250 g of acidic almond lipoprotein, having an active content of 60-80% and obtained as described above, are added. The product is maintained under stirring until the complete dispersion of the latter, and 75 g of a potassium hydrate solution are then slowly added, in small amounts at a time, still under stirring. At the end of the addition, the mixture is stirred for a further 20 minutes and the pH is controlled and kept at a value of 6.5-7.5. The other physico-chemical parameters, which must correspond to the following specification, are also controlled:

| | |
|---|---|
| appearance at 20°C: | waxy mass |
| colour: | ivory white |
| odour: | slightly neutral |
| Gardner colour: | max 3 |
| melting point: | 55-60°C |
| pH value: | 6.5-7.5 |
| proteic nitrogen N₂: | 1.4-1.8% |
| acidity value: | 10-20 |
| OH value: | 160-180 |
| Iodine number: | < 5 |
| heavy metals: | lower than 10 ppm |
| hydroxy proline hypro: | absent |

### EXAMPLE 2

| (preferred) | | |
|---|---|---|
| 1. Palmitoyl Hydrolyzed Rice Protein 75 s.a. | 30% | 10-50% |
| 2. Rice phytosterols (gamma oryzanol) | 10% | 5-30% |
| 3. Glyceryl Stearate | 26.25% | 10-40% |
| 4. Cetearyl Alcohol | 26.25% | 10-40% |
| 5. Potassium Hydroxide 50% | 7.5% | 5-15% |

262.5 g of glyceryl stearate and 262.5 g of cetearyl alcohol are melted at 80°C in a Pyrex glass until the product is completely liquid. 100 g of rice phytosterols are added at the same temperature, and the temperature is then increased to 105°C, the whole mixture is stirred until a complete solution is obtained, it is then cooled, still under stirring, to 80°C. At this point, 300 g of acidic rice lipoprotein, having an active content of 60-80% and obtained as described above, are added. The product is maintained under stirring until the complete dispersion of the latter and 75 g of potassium hydrate are then slowly added, in small amounts at a time, still under stirring. At the end of the addition, the whole mixture is stirred for a further 20 minutes and the pH is controlled and kept at a value of 6.5-7.5. The other physico-chemical parameters, which must correspond to the following specification, are also controlled:

| | |
|---|---|
| appearance at 20°C: | waxy mass |
| colour: | ivory white |
| odour: | slightly neutral |
| Gardner colour: | max 3 |
| melting point: | 52-54°C |
| pH value: | 6.5-7.5 |
| proteic nitrogen N₂: | 1.2-1.6% |
| acidity value: | 10-20 |
| OH value: | 160-180 |
| Iodine number: | < 5 |
| heavy metals: | lower than 10 ppm |
| hydroxy proline hypro: | absent |

### EXAMPLE 3

| (preferred) | | |
|---|---|---|
| 1. Cocoyl hydrolyzed rice protein 75 s.a. | 15% | 10-50% |
| 2. Cocoyl hydrolyzed almond protein 75 s.a. | 15% | 10-50% |
| 3. Brassic phytosterols | 7.5% | 5-20% |
| 4. Rice phytosterols (gamma oryzanol) | 7.5% | 5-20% |
| 5. Glyceryl Stearate | 26.25% | 10-40% |
| 6. Cetearyl Alcohol | 26.25% | 10-40% |
| 7. Sodium Hydroxide 30% | 7.5% | 5-15% |

262.5 g of glyceryl stearate and 262.5 g of cetearyl alcohol are melted at 80°C in a Pyrex glass until the product is completely liquid. 75 g of phytosterols and 75 g of gamma oryzanol are added at the same temperature, and the temperature is then increased to 105°C, the whole mixture is stirred until a complete solution is obtained, it is then cooled, still under stirring, to 80°C. At this point, 150 g of acidic rice lipoprotein and 150 g of almond lipoprotein, having an active titer of 60-80% and obtained as described above, are added. The product is maintained under stirring until the complete dispersion of the two latter products, and 75 g of a solution of potassium hydrate are then slowly added, in small amounts at a time, still under stirring. At the end of the addition, the whole mixture is stirred for a further 20 minutes and the pH is controlled and kept at a value of 6.5-7.5. The other physico-chemical parameters, which must correspond to the following specification, are also controlled:

| | |
|---|---|
| Gardner colour: | max 3 |
| melting point: | 50-55°C |
| pH value: | 6.5-7.5 |
| proteic nitrogen N₂: | 1.2% |
| acidity value: | 10-30 |
| OH value: | 150-190 |
| Iodine number: | <5 |
| heavy metals: | lower than 10 ppm |
| hydroxy proline hypo: | absent |

### EXAMPLE 4

The following composition was prepared according to the procedures described above.

| (preferred) | | |
|---|---|---|
| 1. Palmitoyl hydrolyzed wheat protein | 10-50% | 15% |
| 2. Rice lecithin | 5-15% | 7.5% |
| 3. Soy phosphatidyl choline | 5-15% | 7.5% |
| 4. Brassic phytosterols | 2-10% | 5% |
| 5. Gamma oryzanol | 2-10% | 5% |
| 6. Glyceryl Stearate | 10-40% | 28% |
| 7. Glyceryl Alcohol | 10-40% | 28.25% |
| 8. Sodium Hydroxide 30% | 2-7% | 3.75% |

### EXAMPLE 5

A non-invasive efficacy evaluation was effected with double blanks of two formulations of an anti-age cream, compared with a placebo, using the following products:
Cream 1 containing placebo;
Cream 2 containing wheat lipoprotein (example 4);
Cream 3 containing almond lipoprotein (example 1).

The products, codified as 1, 2 and 3, were applied on the forearms of 15 volunteers (subjects), all females, aged between 44 and 70 years (average age 53 years), under menopause conditions, enlisted in the study on the basis of inclusion/exclusion criteria as provided for in the protocol.

The evaluation was effected with the help of non-invasive skin bio-engineering instruments (t0=0, t1=30 days, t2=60 days, t3=90 days) to back up the clinical evaluation, considering the irritation potential of the products under examination. A utilization test was carried out on the creams 1, 2 and 3, during the clinical evaluation.

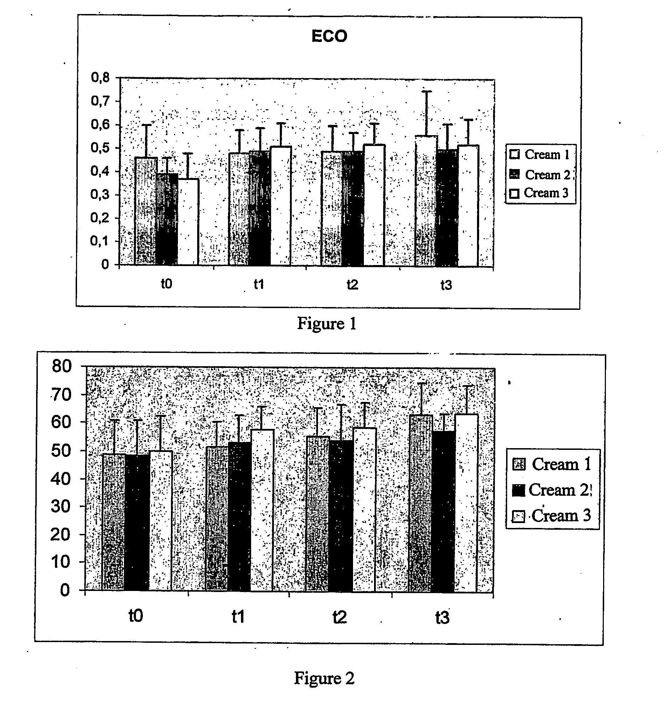

Procedures: evaluation of the irritation potential of the products under examination, applied on 15 healthy volunteers, twice a day, on the left and right forearm, over a period of three months. The volunteers were selected among those who did not have marks or injuries on the forearms, capable of interfering in any way with the results obtained. They were instructed to apply the cream on the right and left forearm, twice a day, interrupting any other hydrating treatment in the same area for the whole period of the examination. The evaluation was effected according to the following methods:
- on the occasion of the two examinations, the volunteers were instructed not to apply the product on the area being tested ;
- the clinical evaluation was effected by considering the skin sensitivity for erythema, desquamation, edema and pruritis on the basis of a clinical score; the results are shown in the diagrams of figures 1 and 2.

Conclusions: no irritating reaction, evaluated according to the clinical parameters of erythema, desquamation, edema and pruritis, was observed during the period of application. All subjects concluded the treatment, applying the products twice a day. None of them interrupted the treatment before its expiration term as a result of intolerance to the products.

The products were applied at random, for a period of three months, on the forearms of the 15 volunteers. 5 of the 15 subjects applied creams 1 and 2, 5 subjects used creams 1 and 3 and 5 subjects creams 2 and 3, according to the following random order:
Subjects 1-7-11 applied cream 1 on the right arm, and cream 2 on the left arm
Subjects 2-8-14 applied cream 1 on the left arm, and cream 3 on the right arm
Subjects 3-9-15 applied cream 2 on the right arm, and cream 3 on the left arm
Subjects 5-13 applied cream 1 on the right arm, and cream 3 on the left arm
Subjects 4-10 applied cream 1 on the left arm, and cream 2 on the right arm
Subjects 6-12 applied cream 2 on the left arm, and cream 3 on the right arm

### 1-HYDRATION

a) The evaluation of the skin hydration was carried out by means of a Corneometer (CM 110 Courage & Khazaka, Germany), an instrument which allows the hydration of the upper layer of the skin to be measured in terms of capacitance. It consists of a probe whose square surface (7x7 mm) covered by a special glass, acts as a sensor capable of moving axially when applied to the skin surface, within a range of at least 3 mm. The instrument measures the capacitance in arbitrary units, which are proportional to the water content of the outer skin layer and therefore to its hydration. This technique is particularly used for detecting the hydration variations between basal and final states, or for having a dynamic evaluation of the hydration state, in this latter case one speaks about a sorption/desorption test (SDT). The test consists in recording the values, at regular intervals of 30 sec. for a total of 120 sec., after water has been applied for 10 seconds; the curve thus obtained expresses the capacity of the outer skin layer of withholding or dissipating water.
b) Results

The results are indicated in table I figure 1 and 2

A modest increase in hydration was observed for creams 1 and 3, after a 3-month treatment (p ≤ 0.05).

### 2. BIO-MECHANICAL PROPERTIES

### a) Method

The evaluation of the bio-mechanical parameters was effected by means of a Cutometer SEM 74 (Courage & Khazaka, Germany). This instrument allows various bio-mechanical parameters of the skin to be measured, such as the skin elasticity degree -R2-, the extendibility degree -R0-, the visco-elastic properties -R6-, which are an expression of the skin tone and are correlated with the functionality of the elastic fibres and collagen present in the derma.

The measurement is effected by applying to the skin surface, an exploring probe capable of creating repeated automatic depressions which produce suctions on a circular area of the skin under examination, and by detecting the elevations through an electronic capacitative system: the skin elasticity reflects the potential recovery capacity on the part of the skin of the original' position, after suction; the extendibility capacity is a more specific measurement of the skin tone; the visco-elastic behaviour gives a measurement of the overall skin quality.

### EXTENDIBILITY R0

### b) Results

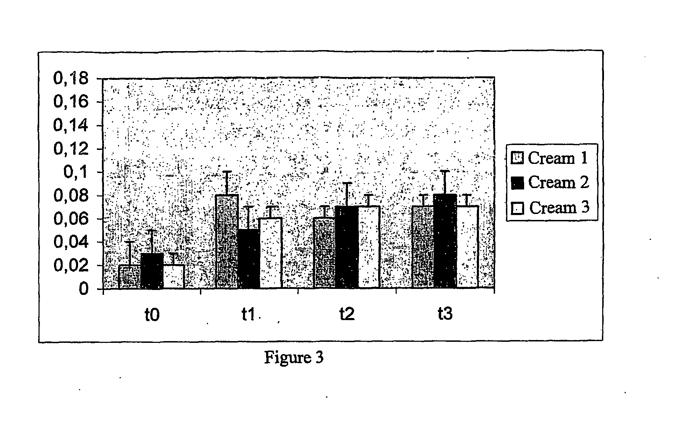

The results are shown in table II-figure 3.

### b) Statistics

A significant increase in the skin extendibility capacity for all creams after a three-month treatment (p-value ≤ 0.0002).

### Elasticity R2

### c) Results

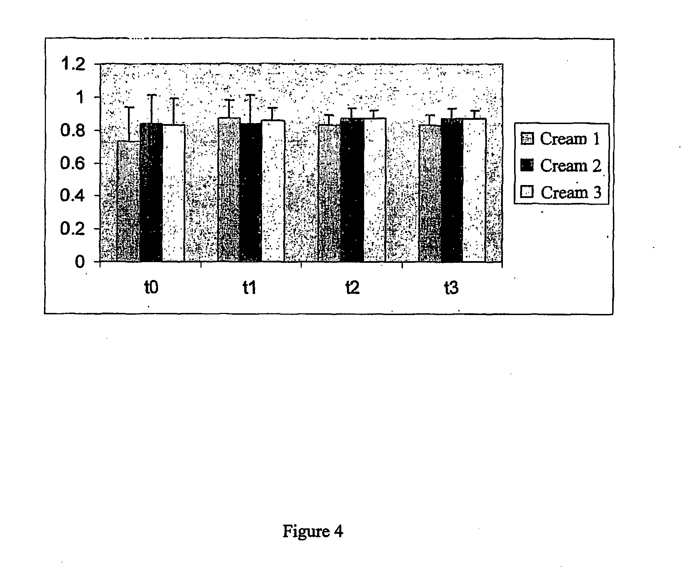

The results are shown in table III - figure 4

### d) Statistics

No increase in the skin elasticity after a three-month treatment.

### Visco-elastic index R6

### e) Results

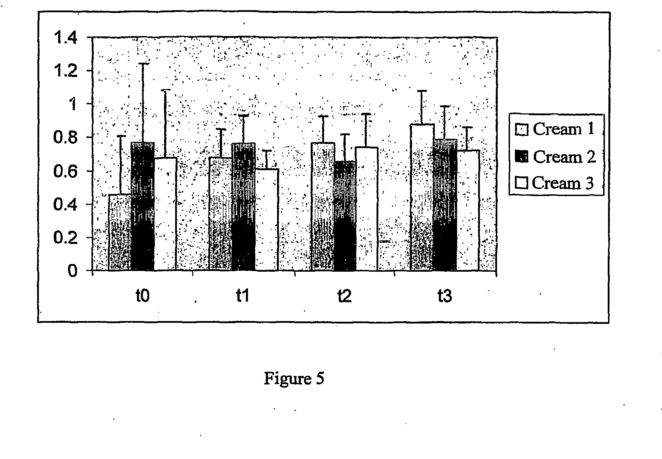

The results are shown in table IV - figure 5

### f) Statistics

A significant increase in the visco-elastic index for cream 1, after a three-month treatment (p=0.03)

### 3- Ultrasounds (ECO)

An evaluation of the skin layer depth is effected by means of ultrasounds. The ultrasound source consists of a probe emitting an ultrasound beam and receives the return signal: when the ultrasound beam passes through structurally different tissues, different echoes are produced, according to the acoustic properties of the tissue; the return echoes are collected by the probe and converted by the transducer into an electric signal which is visualized on a screen as bands. The depth of the outer skin layer is expressed by the corresponding registered band.

### g) Results

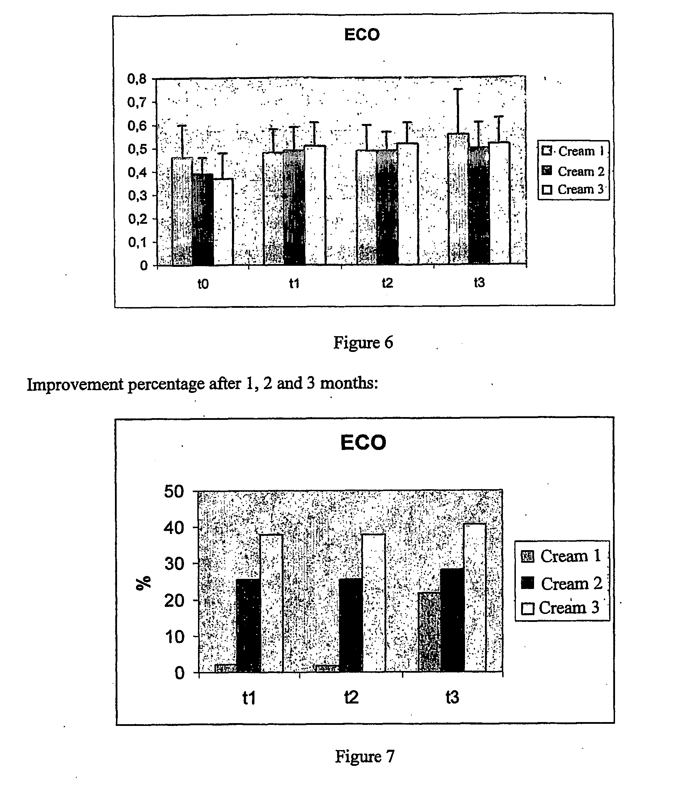

The results are shown in Table V - figures 6 and 7.

### h) Statistics

A significant increase in the skin depth for creams 2 and 3 after 2- and 3-month treatment (p≤ 0.008).

Final conclusions: even though, after three months of treatment, the differences relating to hydration and elasticity were not relevant, and even if an improvement in extendibility was observed for all creams, significant differences (final vs basal with p ≤ 0.008) were observed with creams 2 and 3, in terms of increase in skin depth. These differences were already evident from the second month of treatment. Skin depth is a very important parameter as it relates to the efficacy of anti-age topic products. In particular, it tends to diminish in the skin of a woman in the menopause phase. Our data therefore suggest an important role on the part of the creams defined as 2 and 3, not only in age-resistance, but also in attenuating damage already produced with time.

## Claims

1. A composition of emulsifiers for cosmetic and pharmaceutical products comprising:
(a) a first component selected from the group consisting of lipoproteins of almond, rice, wheat, said lipoproteins being the reaction product of fatty acids with hydrolysed proteins, and
(b) a second component selected from the group consisting of phytosterols and vegetable phospholipids.

2. A composition according to claim 1, wherein the phytosterols are selected from the group consisting of soybean phytosterol, rape phytosterol, rice phytosterol and brassic phytosterol.

3. A composition according to claim 1, wherein vegetable phospholipids are selected from the group consisting of soybean phospholipids and rice phospholipids.

4. A composition according to claim 1, comprising at least a further substance selected from vegetable monoglycerides and fatty alcohols.

5. Use of a composition according to claim 1, for the manufacture of emulsions and creams for body and skin care.

6. Use of an emulsifier composition according to claim 1 for the manufacture of emulsions and creams for protecting the skin against outside exogenous aggression and solar UVB radiations.

7. Use of an emulsifier composition according to claim 1 for protecting the skin against ageing.

## Patentansprüche

1. Zusammensetzung von Emulgiermitteln für kosmetische und pharmazeutische Produkte umfassend:
(a) eine erste Komponente ausgewählt aus der Gruppe bestehend aus Lipoproteinen von Mandel, Reis, Weizen, wobei die Lipoproteine das Reaktionsprodukt von Fettsäuren mit hydrolysierten Proteinen sind, und
(b) eine zweite Komponente ausgewählt aus der Gruppe bestehend aus Phytosterolen und pflanzlichen Phospholipiden.

2. Zusammensetzung nach Anspruch 1, wobei die Phytosterole ausgewählt sind aus der Gruppe bestehend aus Sojabohnen-Phytosterol, Raps-Phytosterol, Reis-Phytosterol und Brassica-Phytosterol (brassic phytosterol).

3. Zusammensetzung nach Anspruch 1, wobei pfanzliche Phospholipide ausgewählt sind aus der Gruppe bestehend aus Sojabohnen-Phospholipiden und Reis-Phospholipiden

4. Zusammensetzung nach Anspruch 1, umfassend wenigstens eine weitere Substanz, ausgewählt aus pflanzlichen Monoglyceriden und Fettalkoholen.

5. Verwendung einer Zusammensetzung nach Anspruch 1 für die Herstellung von Emulsionen und Cremes für Körper- und Hautpflege.

6. Verwendung einer Emulgiermittelzusammensetzung nach Anspruch 1 für die Herstellung von Emulsionen und Cremes zum Schützen der Haut gegen einen von außen kommenden exogenen Angriff und solare UVB-Strahlung.

7. Verwendung einer Emulgiermittelzusammensetzung nach Anspruch 1 zum Schützen der Haut vor dem Altern.

## Revendications

1. Composition d'émulsifiants pour des produits cosmétiques et pharmaceutiques comprenant :
a) un premier composant choisi dans le groupe constitué par les lipoprotéines d'amande, de riz, de blé, lesdites lipoprotéines étant le produit de réaction d'acides gras avec des protéines hydrolysées, et
b) un second composant choisi dans le groupe constitué par les phytostérols et les phospholipides végétaux

2. Composition selon la revendication 1, dans laquelle les phytostérols sont choisis dans le groupe constitué par le phyrostérol de soja, le phytostérol de colza, le phytostérol de riz et le phytostérol brassique.

3. Composition selon la revendication 1, dans laquelle les phospholipides végétaux sont choisis dans le groupe constitué par les phospholipides de soja et les phospholipides de riz.

4. Composition selon la revendication 1, comprenant au moins une substance supplémentaire choisie parmi les monoglycérides végétaux et les alcools gras.

5. Utilisation d'une composition selon la revendication 1, pour la fabrication d'émulsions et de crèmes pour le soin du corps et de la peau.

6. Utilisation d'une composition d'émulsifiants selon la revendication 1, pour la fabrication d'émulsions et de crèmes pour la protection de la peau contre l'agression exogène de l'extérieur et les rayonnements solaires UVB.

7. Utilisation d'une composition d'émulsifiants selon la revendication 1, pour la protection de la peau contre le vieillissement.
